# EUROPÄISCHE PATENTSCHRIFT

(11) **EP 0 574 799 B1**
(45) Veröffentlichungstag und Bekanntmachung des Hinweises auf die Patenterteilung: **17.09.1997**
(21) Anmeldenummer: 93109173.0
(22) Anmeldetag: 08.06.1993
(51) Int. Cl.: C09B 62/04, C09B 62/503, C07C 233/43

(54) **Verfahren zur Herstellung von faserreaktiven Formazanfarbstoffen sowie Aminophenole**
Method for the preparation of fibre-reactive formazana dyes as well as aminophenols
Procédé préparation de colorants de type formazane réactifs sur la fibre et aminophénols

(30) Priorität: 13.06.1992 DE 4219421
(43) Veröffentlichungstag der Anmeldung: 22.12.1993
(73) Patentinhaber: BASF Aktiengesellschaft, 67063 Ludwigshafen (DE)
(72) Erfinder: Marschner, Claus, Dr., W-6720 Speyer (DE); Patsch, Manfred, Dr., W-6704 Wachenheim (DE)

(56) Entgegenhaltungen:
- EP-A- 0 315 046
- DE-A- 3 607 825
- DE-A- 4 142 132
- T.W.GREENE et al. "Protective Groups in Organic Synthesis", 2nd Auflage, John Wiley & Sons Inc., 1991, Seiten 349-352

## Beschreibung

Die vorliegende Erfindung betrifft ein neues Verfahren zur Herstellung von faserreaktiven Formazanfarbstoffen durch Diazotierung einer geschützten Hydroxyphenylendiaminverbindung und Kupplung mit einer Hydrazonverbindung, Metallisierung, Abspaltung der Schutzgruppe, Umsetzung mit einem Halogentriazin und gegebenenfalls anschließend mit einem Amin oder einer Hydroxyverbindung. Die vorliegende Erfindung betrifft trifft weiterhin neue Aminophenole.

Aus der EP-A-280 139 ist bereits bekannt, Aminoformazanfarbstoffe durch Umsetzung von einem Arylhydrazon mit einem diazotierten o-Hydroxyaminobenzol herzustellen. Im Endprodukt vorhandene Aminogruppen müssen dabei vor der Reaktion mittels Acetylierung geschützt und die Acetylschutzgruppe anschließend wieder abgespalten werden.

Für die alkalische Hydrolyse der Schutzgruppe werden jedoch hohe Laugenkonzentrationen, hohe Reaktionstemperaturen und lange Reaktionszeiten benötigt.

Nachteilig an den bekannten Verfahren ist weiterhin, daß vor der Umsetzung mit hydrolyseempfindlichen Ankerkomponenten, z.B. mit Halogentriazinen, die alkalische Lösung zunächst neutralisiert werden muß, wodurch erhebliche Mengen an Salz entstehen.

Aufgabe der vorliegenden Erfindung war es deshalb, ein ökonomisch und ökologisch verbessertes Herstellungsverfahren für Aminoformazanverbindungen bereitzustellen, das die genannten Probleme vermeidet und die Synthese von Aminoformazanverbindungen und deren weitere Umsetzung zu Reaktivfarbstoffen in hoher Raum-Zeit-Ausbeute ermöglicht.

Es wurde nun gefunden, daß die Herstellung von faserreaktiven Formazanfarbstoffen, die in Form der freien Säure der Formel I 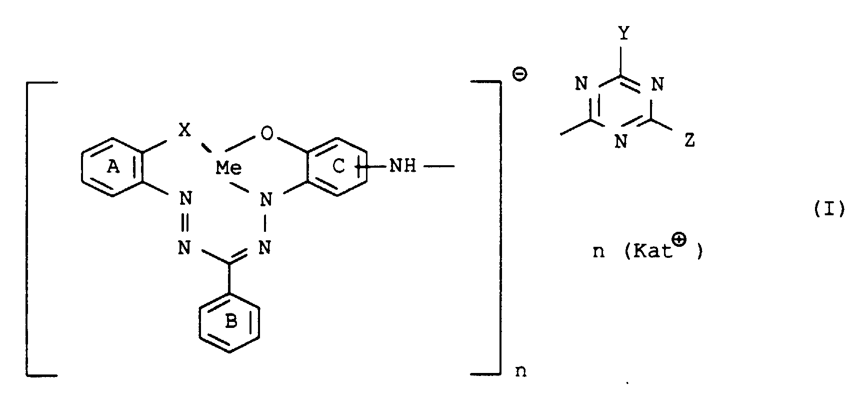 entsprechen, in der
- Kat^{⊕}: das Äquivalent eines Kations,
- n: 1 oder 2,
- X: Sauerstoff oder ein Rest der Formel CO-O oder SO₂-O,
- Me: Kupfer oder Nickel,
- Y: Halogen und
- Z,: wenn n 1 ist, Halogen, C₁-C₄-Alkoxy, Amino, C₁-C₄-Mono- oder Dialkylamino, substituiertes C₂-C₄-Alkylamino oder Phenyl- oder N-(C₁-C₄-Alkyl)-N-phenylamino, wobei die Phenylgruppe jeweils substituiert sein kann, oder, wenn n 2 ist, einen Rest der Formel 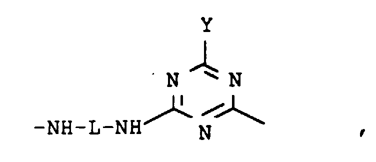 worin L für C₂-C₆-Alkylen oder gegebenenfalls durch Hydroxysulfonyl substituiertes Phenylen steht und Y die oben genannte Bedeutung besitzt, bedeuten und
die Ringe A, B und C weitere Substituenten tragen können,
vorteilhaft gelingt, wenn man
a) in einem 1. Schritt ein Aminophenol der Formel II 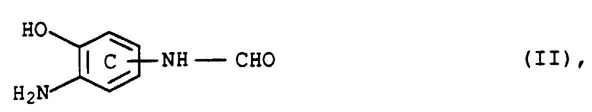 in der der Ring C die obengenannte Bedeutung besitzt, diazotiert, mit einer Kupplungskomponente der Formel III 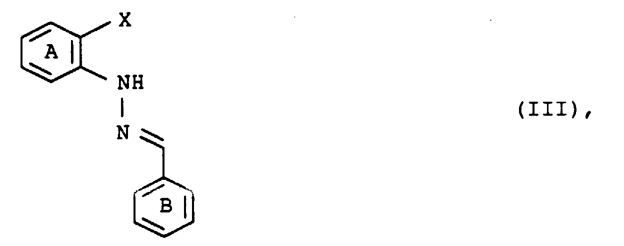 in der X und die Ringe A und B jeweils die obengenannte Bedeutung besitzen, kuppelt und vor, während oder nach der Kupplungsreaktion ein Kupfer- oder Nickelsalz zusetzt,
b) in einem 2. Schritt die resultierende Formazanverbindung der Formel IV 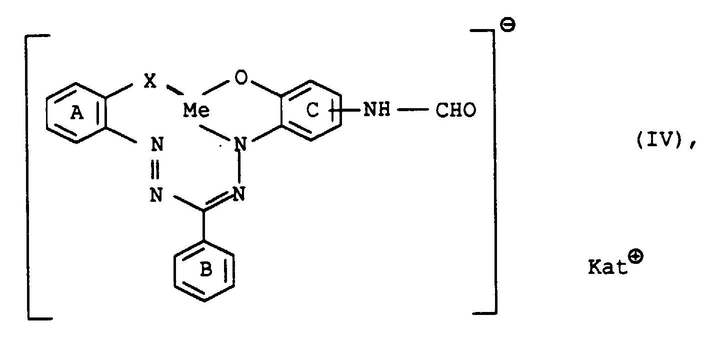 in der Kat^{⊕} , X, Me und die Ringe A, B und C jeweils die obengenannte Bedeutung besitzen, säure- oder basenkatalysiert lysiert verseift und
c) in einem 3. Schritt die resultierende Aminoverbindung mit einem Halogentriazin der Formel Va oder Vb 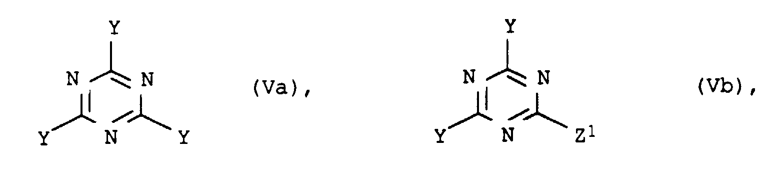 wobei Y jeweils die obengenannte Bedeutung besitzt und Z¹ C₁-C₄-Alkoxy, Amino, C₁-C₄-Mono- oder Dialkylamino, substituiertes C₂-C₄-Alkylamino oder Phenyl- oder N-(C₁-C₄-Alkyl)-N-phenylamino, wobei die Phenylgruppe jeweils substituiert sein kann, oder einen Rest der Formel 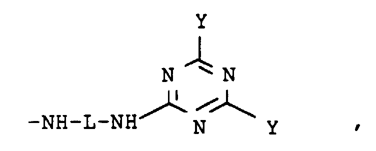 worin L und Y jeweils die obengenannte Bedeutung besitzen, bedeutet, zur Reaktion bringt.

Kat^{⊕} stellt des Äquivalent eines Kations dar. Es stellt entweder ein Proton dar oder leitet sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl- Tetraalkyl oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch Sauerstoffatome in Etherfunktion unterbrochen sein kann.

Besonders als Kationen hervorzuheben sind Protonen oder Lithium-, Natrium oder Kaliumionen.

Wenn die Ringe A, B und/oder C in Formel I weitere Substituenten tragen, so können dabei z.B. Halogen, dabei insbesondere Chlor oder Brom, oder Hydroxysulfonyl in Betracht kommen. Die Ringe A, B und/oder C weisen dann in der Regel einen oder zwei solcher Substituenten auf.

Wenn Z in Formel I substituiertes Phenylamino bedeutet, so können dabei als Substituenten z.B. Hydroxysulfonyl, 2-Sulfatoethylsulfonyl, 2-Chlorethylsulfonyl, 3-(2-Sulfatoethylsulfonyl)propanoylamino, 3-(2-Chlorethylsulfonyl)propanoylamino, 4-(2-Sulfatoethylsulfonyl)butyrylamino oder 4-(2-Chlorethylsulfonyl)butyrylamino in Betracht kommen. Der Phenylring ist dann in der Regel ein- oder zweifach substituiert.

Wenn Z in der Formel I substituiertes C₂-C₄-Alkylamino bedeutet, so können dabei als Substituenten z.B. Hydroxysulfonyl, 2-Sulfatoethylsulfonyl oder 2-Chlorethylsulfonyl in Betracht kommen.

Reste Y und Z sind z.B. Fluor, Chlor oder Brom.

Reste Z sind weiterhin z.B. Methoxy, Ethoxy, Propoxy, Isopropoxy, Butoxy, Isobutoxy, sec-Butoxy, Mono- oder Dimethylamino, Mono- oder Diethylamino, Mono- oder Dipropylamino, Mono- oder Diisopropylamino, Mono- oder Dibutylamino, N-Methyl-N-ethylamino, 2-Hydroxysulfonylethylamino, 2-Chlorethylsulfonylamino, Phenylamino, 2-, 3- oder 4-Hydroxysulfonylphenylamino, 3- oder 4-(2-Sulfatoethylsulfonyl)phenylamino, 3- oder 4-(2-Chlorethylsulfonyl)phenylamino, N-Methyl- oder N-Ethyl-N-[4-(2-sulfatoethylsulfonyl)phenyl]amino, 3- oder 4-[4-(2-Sulfatoethylsulfonyl)butyrylamino]phenylamino oder 3- oder 4-[4-(2-Chlorethylsulfonyl)butyrylamino]phenylamino.

Reste L sind z.B. (CH₂)₂, (CH₂)₃, (CH₂)₄, (CH₂)₅, (CH₂)₆, CH(CH₃)CH₂, CH(CH₃)CH(CH₃), 1,2-, 1,3- oder 1,4-Phenylen oder 4-Hydroxysulfonyl-1,3-phenylen.

Geeignete Kupfer- oder Nickelsalze, die im 1. Reaktionsschritt zugesetzt werden, sind in der Regel zweiwertige Salze, wie Kupfersulfat, Kupferchlorid, Kupferacetat, Nikkelchlorid, Nickelsulfat oder Nickelacetat.

Das erfindungsgemäße Verfahren wird zweckmäßig so durchgeführt, daß man das Aminophenol II in einem 1. Schritt auf an sich bekannte Weise diazotiert, z.B. in wäßrigem Medium, mit Natrium- oder Kaliumnitrit in Gegenwart einer Mineralsäure, beispielsweise Salzsäure oder Schwefelsäure, bei einer Temperatur von 0 bis 10°C, oder in einem organischen Lösungsmittel mit Salpetrigsäureestern von Glykolen als Diazotierungsreagenz bei einer Temperatur von 0 bis 10°C. Das resultierende Diazoniumsalz wird dann, üblicherweise als Lösung oder Suspension, zu der Kupplungskomponente III in wäßrigem Medium gegeben. Die Kupplung wird in der Regel bei einer Temperatur von 10 bis 25°C und einem pH-Wert von 6 bis 10 vorgenommen. Vor, während oder nach der Kupplungsreaktion wird dem Reaktionsgemisch noch ein Kupfer- oder Nickelsalz zugesetzt.

Nach einer Nachrührphase von ca. 1 bis 4 Stunden bei einer Temperatur von 10 bis 25°C ist die Bildung des Formazans IV beendet.

Je mol Aminophenol II wendet man im allgemeinen 1,0 bis 1,1 mol Diazotierungsreagenz, 0,9 bis 1,1 mol Kupplungskomponente III und 0,9 bis 1,2 mol Kupfer- oder Nickelsalz an.

Das Formazan IV kann entweder zwischenisoliert (z.B. mittels Aussalzens) oder direkt in der Reaktionslösung säure- oder basenkatalysiert verseift werden.

Der 2. Schritt der säurekatalysierten Verseifung wird in der Regel bei einem pH-Wert von 1 bis 3, vorzugsweise 1 bis 2, und bei einer Temperatur von 40 bis 80°C in wäßrigem Milieu vorgenommen. Als Säuren kann man z.B. Mineralsäure, wie Salzsäure, Schwefelsäure oder Phosphorsäure, verwenden. Je mol Formazan IV wendet man in der Regel ca. 2 Moläquivalent Säure an.

Die basenkatalysierte Verseifung wird in der Regel bei einem pH-Wert von 8 bis 12, vorzugsweise 10 bis 11, und bei einer Temperatur von 40 bis 80°C in wäßrigem Milieu vorgenommen. Als Basen kann man z.B. wäßrige Lösungen (1 bis 20 gew.-%ig) von Lithium-, Natrium- oder Kaliumhydroxid verwenden. Je mol Formazan IV wendet man in der Regel 3 bis 12 Moläquivalent Base an.

Nach einer Dauer von 0,5 bis 2 Stunden ist die Verseifung beendet.

Im folgenden 3. Schritt erfolgt die Umsetzung der resultierenden Aminoverbindung der Formel IVa 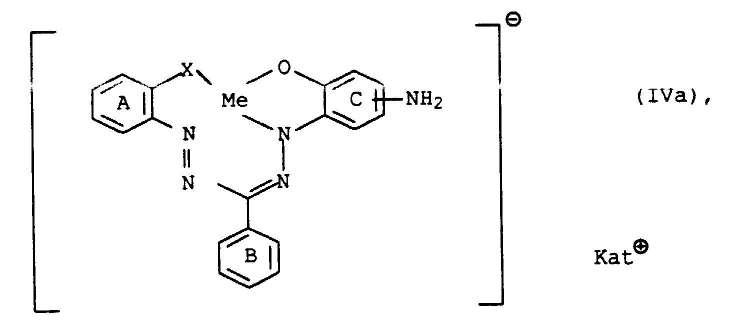 in der Kat^{⊕}, X, Me und die Ringe A, B und C jeweils die obengenannte Bedeutung besitzen, mit dem Triazin der Formel Va oder Vb. Dazu wird die Reaktionslösung auf Raumtemperatur abgekühlt und auf einen pH-Wert von 4 bis 6 eingestellt. Dann erfolgt die Zugabe des Triazins Va oder Vb. Der pH-Wert des Reaktionsgemisches wird dabei, beispielsweise durch Zugabe von Natriumhydrogencarbonat, bei 4 bis 4,5 und die Temperatur bei 0 bis 25°C gehalten.

Zur Herstellung derjenigen Verbindungen der Formel I, in der n 2 bedeutet, wird die Aminoverbindung der Formel IVa mit dem Triazin der Formel Vb, worin Z¹ für den obengenannten Rest der Formel 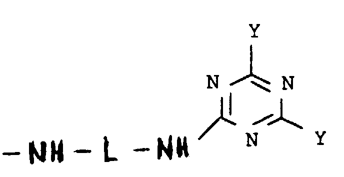 steht, im Molverhältnis von 1,9:1 bis 2,1:1 umgesetzt.

Wenn die Umsetzung mit dem Triazin der Formel Va erfolgt, ist es auch möglich, eine Folgereaktion mit einer Verbindung der Formel VI

Z²-H (VI)

anzuschließen, in der Z² C₁-C₄-Alkoxy, Amino, C₁-C₄-Mono- oder Dialkylamino, substituiertes C₂-C₄-Alkylamino oder Phenyl- oder N-(C₁-C₄-Alkyl)-N-phenylamino, wobei die Phenylgruppe jeweils substituiert sein kann, bedeutet. Zu diesem Zweck wird das Reaktionsgemisch mit 1,0 bis 1,2 mol der Verbindung VI, bezogen auf 1 mol Triazin Va versetzt und bei einer Temperatur von 40 bis 60°C und einem pH-Wert von 5,0 bis 6,5, der durch Zugabe beispielsweise von Natriumhydrogencarbonat eingestellt werden kann, 1 bis 8 Stunden nachgerührt.

Nach beendeter Umsetzung kann der Farbstoff auf an sich bekannte Weise, z.B. durch Sprühtrocknen oder Aussalzen isoliert werden.

Bevorzugt wird das Verfahren zur Herstellung von Formazanfarbstoffen der Formel I angewendet, in der zwei der Ringe A, B und C jeweils einfach durch Hydroxysulfonyl substituiert sind.

Weiterhin bevorzugt wird das Verfahren zur Herstellung von Formazanfarbstoffen der Formel I angewandt, in der Me Kupfer bedeutet.

Weiterhin bevorzugt wird das Verfahren zur Herstellung von Formazanfarbstoffen der Formel I, in der Y Fluor oder Chlor, wobei Chlor besonders zu nennen ist, angewandt.

Ein weiterer Gegenstand der vorliegenden Erfindung sind Aminophenole der Formel II 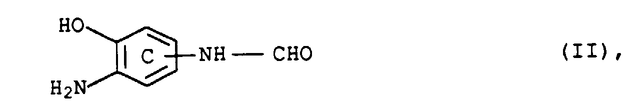 in der der Ring C substituiert sein kann. Geeignete Substituenten sind beispielsweise die oben beispielhaft aufgeführten Reste.

Wie aus den obigen Ausführungen ersichtlich, sind die Aminophenole II wertvolle Zwischenprodukte für das erfindungsgemäße Verfahren.

Ihre Herstellung erfolgt nach an sich bekannten Methoden. Beispielsweise kann man ein Nitrophenol der Formel IIa 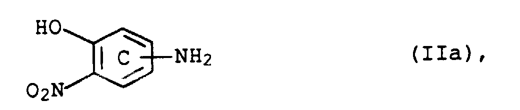 in der der Ring C die obengenannte Bedeutung besitzt, mit Ameisensäure in wäßrigem Milieu formylieren und anschließend die Nitrogruppe katalytisch reduzieren.

Je mol Nitrophenol IIa wendet man zur Formylierung in der Regel 1 bis 15 mol Ameisensäure an. Die Formylierung wird dabei im allgemeinen bei einem pH-Wert von 1 bis 5, vorzugsweise 2 bis 3, und einer Temperatur von 30 bis 80°C, vorzugsweise 35 bis 50°C, vorgenommen.

Die weiteren im erfindungsgemäßen Verfahren zur Anwendung kommenden Produkte sind an sich bekannt und beispielsweise im eingangs genannten Stand der Technik beschrieben.

Der Vorteil des neuen Verfahrens ist darin zu sehen, daß die Formazanfarbstoffe der Formel I auf einfache Weise und in hoher Reinheit und hoher Raum-Zeit-Ausbeute hergestellt werden können. Verglichen mit der Hydrolyse der Acetylschutzgruppe, (siehe z.B. EP-A-0 315 046), die nur sehr schwierig zu vollziehen ist, läßt sich die Formylgruppe sowohl mittels sauer als auch alkalisch katalysierter Hydrolyse unter sehr milden Bedingungen abspalten.

Die Formazanfarbstoffe der Formel I sind wertvolle Reaktivfarbstoffe, die sich in vorteilhafter Weise zum Färben oder Bedrucken von Hydroxylgruppen oder Stickstoffatome aufweisenden organischen Substraten eignen. Solche Substrate sind beispielsweise Leder oder Fasermaterial, das überwiegend natürliche oder synthetische Polyamide oder natürliche oder regenerierte Cellulose enthält. Vorzugsweise eignen sich die mittels des neuen Verfahrens hergestellten Farbstoffe zum Färben und Bedrucken von Textilmaterial auf der Basis von Baumwolle.

Die folgenden Beispiele sollen die Erfindung näher erläutern.

### Beispiel 1

a) 104,4 g 2-Formylamino-6-aminophenol-4-sulfonsäure wurden in 500 ml Wasser gelöst und der pH-Wert mit konz. Salzsäure auf 2 bis 2,3 gestellt. Anschließend wurde bei einer Temperatur von 0 bis 5°C durch Zugabe von 128 g 23 gew.-%iger wäßriger Natriumnitritlösung diazotiert. Durch Zugabe von 2 g Amidosulfonsäure wurde dann überschüssiges Nitrit zerstört.
   Die resultierende Diazosuspension ließ man bei 10 bis 15°C zu einer auf einen pH-Wert von 6,5 bis 7,0 gestellten Mischung aus 144,0 g des Hydrazons der Formel 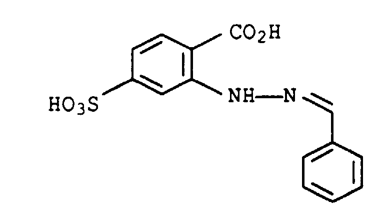 und 117,6 g Kupfersulfat · 5 H₂O in 590 ml Wasser laufen, wobei der pH-Wert zwischen 6,5 und 7,0 gehalten wurde. Nach Beendigung des Zulaufs ließ man noch 2 Stunden bei 15 bis 20°C nachrühren und gab anschließend 300 g Kochsalz zu. Das ausgesalzene Produkt wurde abfiltriert und bei 50°C unter vermindertem Druck getrocknet. Man erhielt 400 g einer elektrolythaltigen Verbindung der Formel 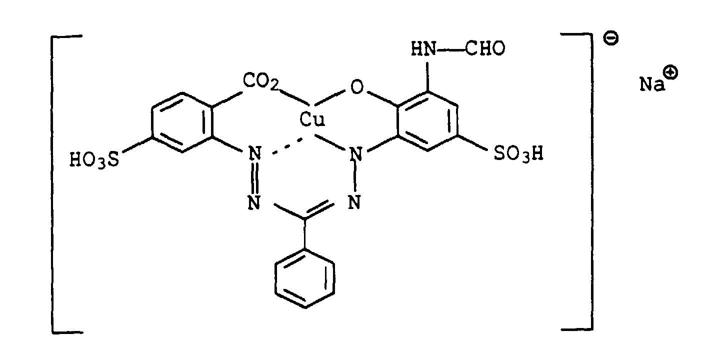 (λₘₐₓ: 646 nm)
b1) 50 g der unter a) erhaltenen Verbindung wurden in 250 ml Wasser gelöst. Der pH-Wert wurde mit konz. Salzsäure auf den Wert 1 gestellt, anschließend wurde auf 40 bis 45°C erwärmt und bei dieser Temperatur bis zur vollständigen Abspaltung der Formylschutzgruppe gerührt (Reaktionskontrolle mittels DC), was etwa 4 Stunden in Anspruch nahm.
   Nach dem Abkühlen auf Raumtemperatur wurde mit Natriumcarbonat ein pH-Wert von 6,0 bis 6,5 eingestellt und das Produkt durch Zugabe von 100 g Kochsalz ausgesalzen. Nach dem Trocknen erhielt man 61 g der elektrolythaltigen Verbindung der Formel 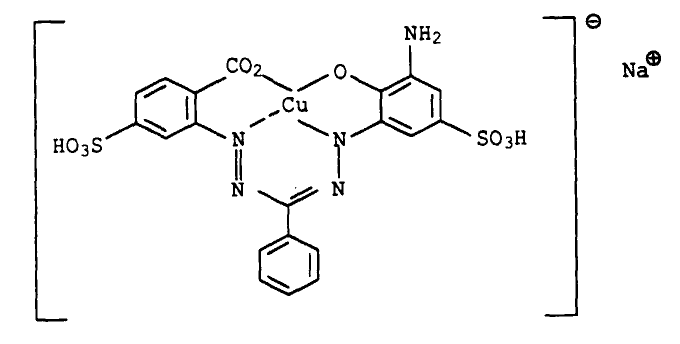
b2) 50 g der unter a) erhaltenen Verbindung wurden in 250 ml 5 gew.-%iger Natronlauge gelöst und auf 60°C erwärmt. Es wurde 30 Minuten bei dieser Temperatur nachgerührt (Reaktionskontrolle mittels DC zeigte dann die vollständige Abspaltung der Schutzgruppe an). Dann wurde auf Raumtemperatur abgekühlt und der pH-Wert mit konz. Salzsäure auf 6,0 bis 6,5 gestellt. Nach dem Aussalzen, Abfiltrieren und Trocknen unter vermindertem Druck bei 50°C erhielt man 63 g der elektrolythaltigen Verbindung der Formel 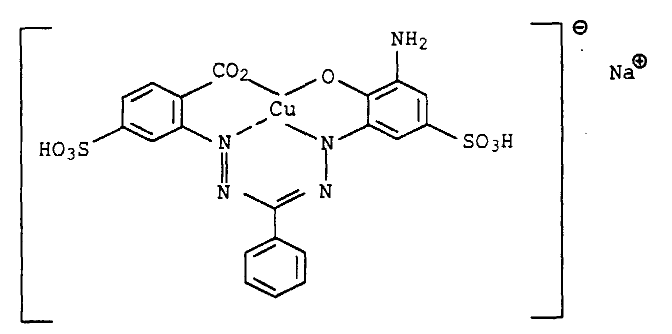
In analoger Weise werden die in der folgenden Tabelle 1 aufgeführten Formazane erhalten.

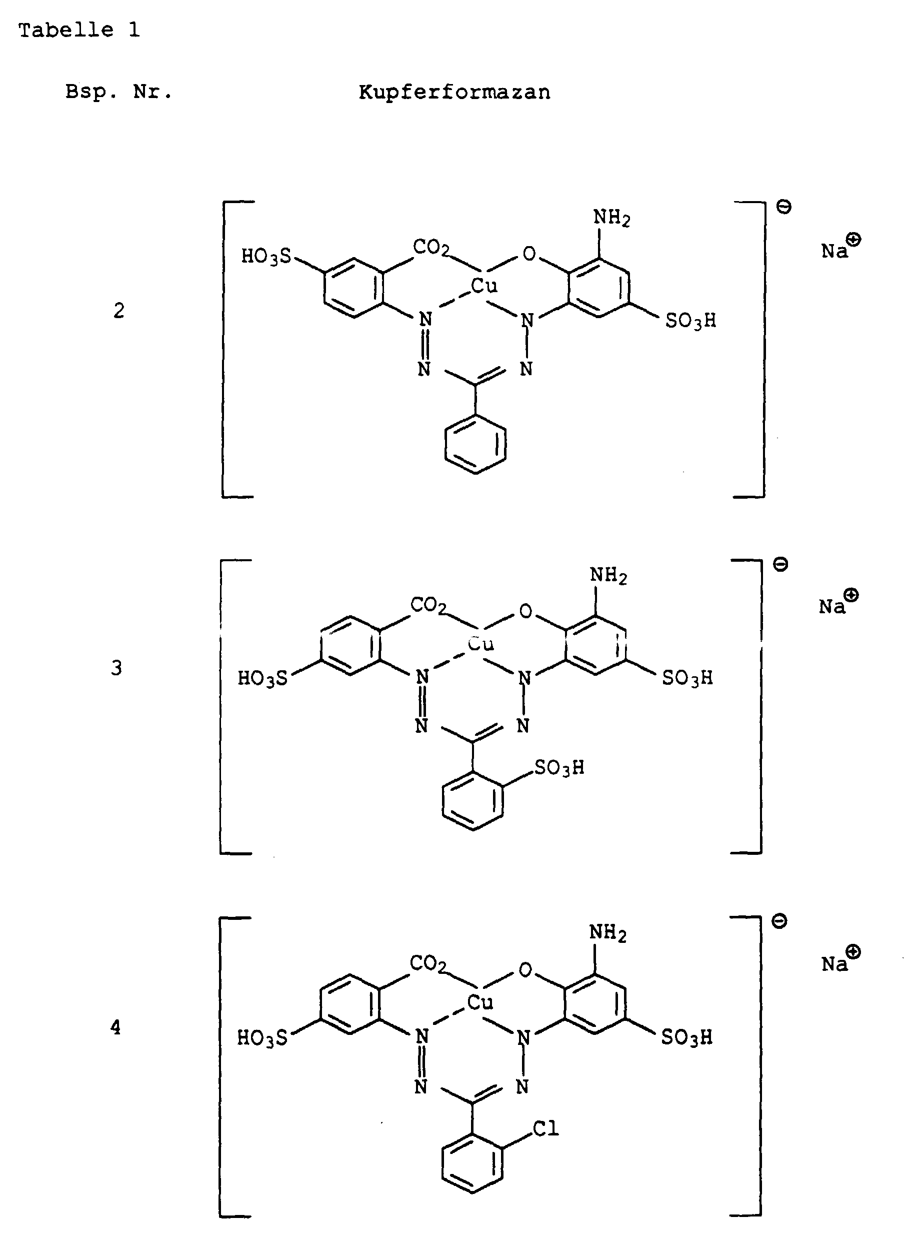

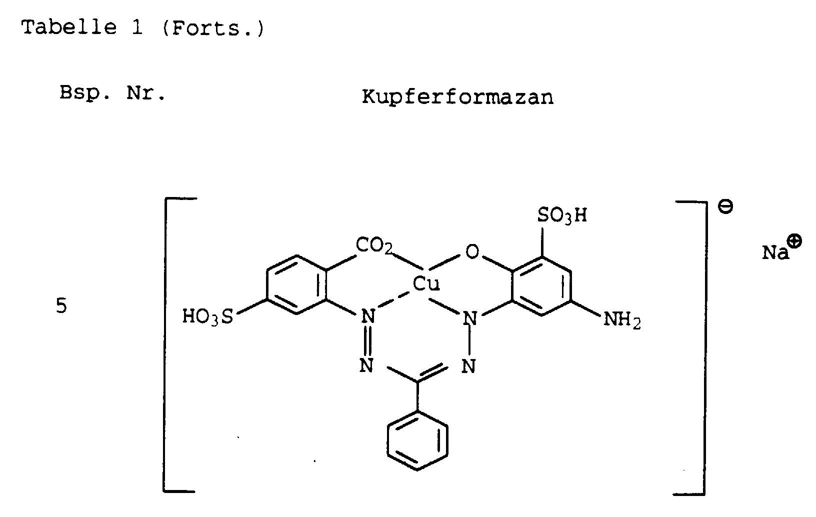

### Beispiel 6

Die in Beispiel 1a) vor dem Aussalzen erhaltene Reaktionslösung wurde mit konz. Salzsäure auf einen pH-Wert von 1 gestellt und anschließend auf 40 bis 45°C erwärmt. Es wurde 4 Stunden bei dieser Temperatur nachgerührt, anschließend wurde der pH-Wert mit festem Natriumcarbonat auf 4,0 bis 4,5 erhöht.

Nach dem Abkühlen auf Raumtemperatur wurden 83,0 g Cyanurchlorid zugegeben. Der pH-Wert des Reaktionsgemisches wurde bis zum Ende der Acylierung durch Zugabe von Natriumhydrogencarbonat bei 4,0 bis 4,5 gehalten. Nach 2 Stunden wurden 80 g 3-Aminobenzolsulfonsäure, gelöst in 150 ml Wasser, eingetragen. Die Suspension wurde auf 40°C erwärmt und der pH-Wert durch Zusatz von Natriumhydrogencarbonat bei 6,0 bis 6,5 gehalten.

Nach 6 Stunden wurde der Formazanfarbstoff der Formel 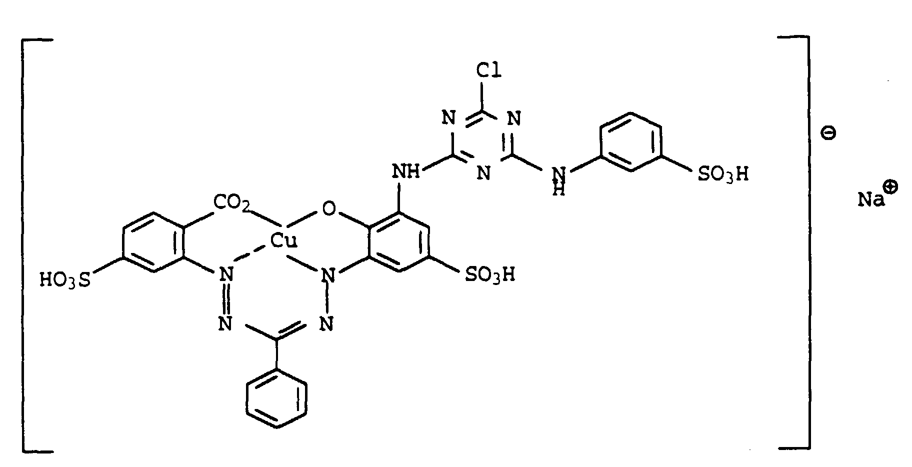 durch Aussalzen isoliert.

In analoger Weise werden die in der folgenden Tabelle 2 aufgeführten Formazanfarbstoffe der Formel 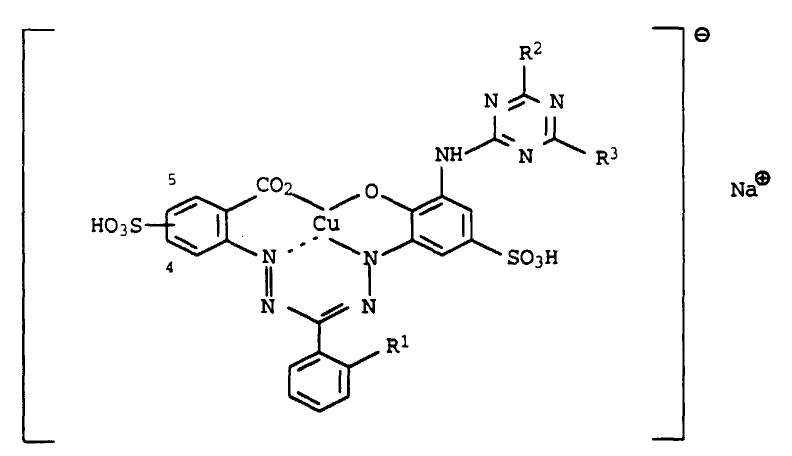 erhalten.

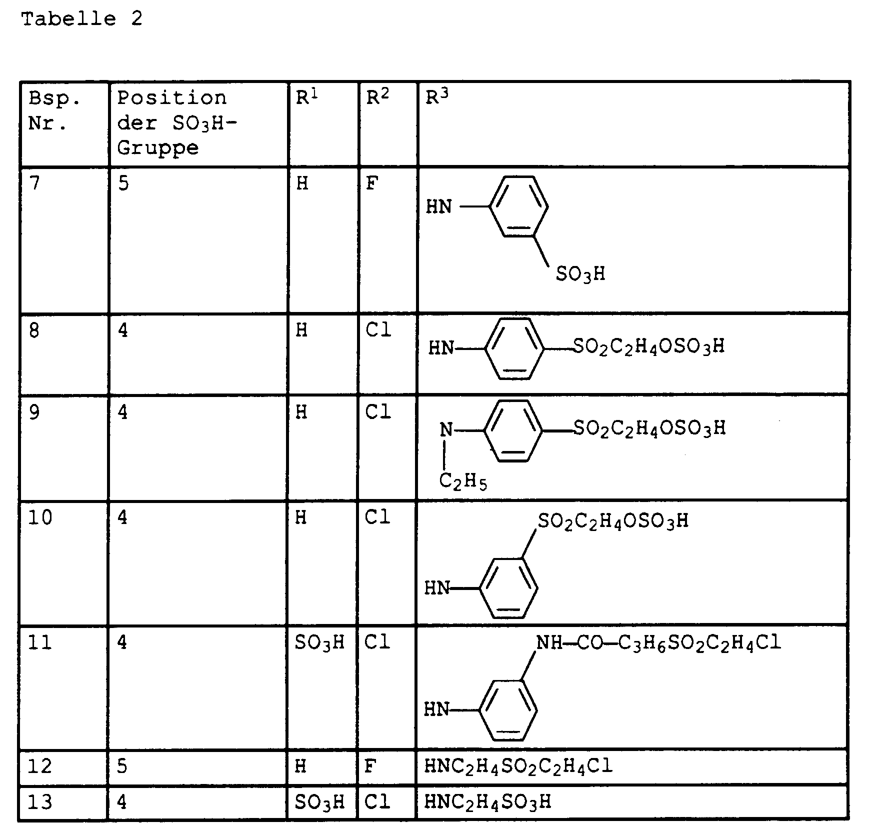

### Beispiel 14

Beispiel 6 wurde wiederholt, jedoch wurden anstelle von 3-Aminobenzolsulfonsäure 45,5 g 4-Amino-2-methylaminobenzolsulfonsäure, gelöst in 100 ml Wasser, zugegeben.

Die Suspension wurde auf 60°C erwärmt und der pH-Wert durch Zusatz von Natriumhydrogencarbonat bei 7,0 bis 7,5 gehalten. Nach 6 Stunden wurde der elektrolythaltige Formazanfarbstoff, der in Form der freien Säure der folgenden Formel entspricht, durch Sprühtrocknen isoliert:

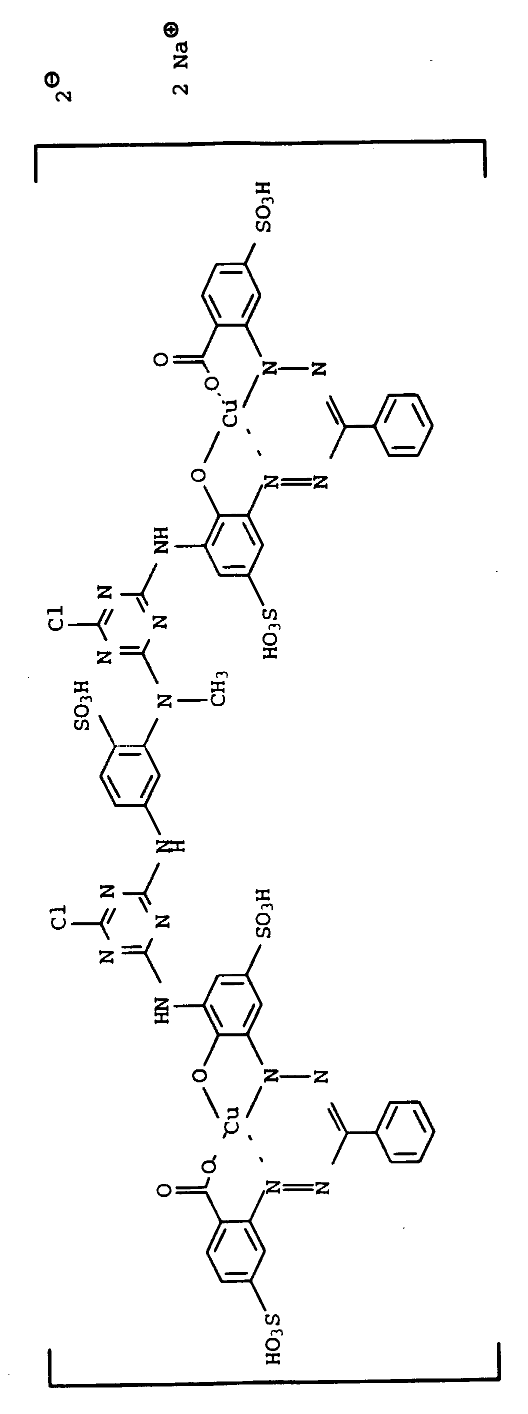

### Beispiel 15

a) 233,5 g 2-Amino-6-nitrophenol-4-sulfonsäure wurden in 1 l Wasser bei 60°C gelöst. Mit Natriumcarbonat wurde ein pH-Wert von 2 eingestellt. Dann wurden innerhalb von 30 Minuten 234,7 g Ameisensäure zugetropft, wobei durch Zugabe von 50 gew.-%iger Natronlauge der pH-Wert bei 2 gehalten wurde.
   Es wurde 4 Stunden bei 60°C nachgerührt. Dann wurde auf 0 bis 5°C abgekühlt, der pH-Wert mit 50 gew.-%iger Natronlauge auf 6,8 erhöht, und das ausgefallene Produkt abgesaugt. Nach dem Trocknen unter vermindertem Druck bei 60°C wurden 466,8 g elektrolythaltiger Verbindung der Formel 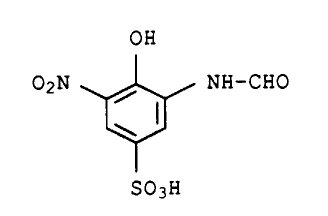 erhalten (Reinheit gemäß HPLC: 97 %)
   ¹H-NMR [d⁶-DMSO] : δ = 7,9, 8,4 (Aromaten-H, CHO), 9,5 (NH) ppm.
b) 465 g der unter a) erhaltenen formylierten Nitroverbindung wurden in 1,4 l Wasser suspendiert und nach Zugabe von 15 g Raney-Nickel bei Raumtemperatur mit Wasserstoff hydriert (Wasserstoffdruck: 2 bar). Nach beendeter Wasserstoffaufnahme wurde vom Katalysator abfiltriert und die Mutterlauge vom Lösungsmittel befreit. Es verblieben 460 g elektrolythaltiger Verbindung der Formel 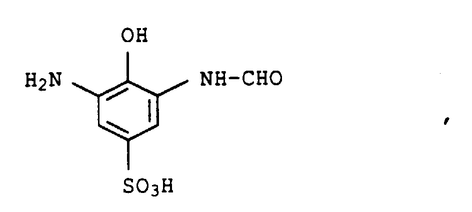 die ohne weitere Reinigung zu Farbstoffsynthesen verwendet werden kann.

## Patentansprüche

1. Verfahren zur Herstellung von faserreaktiven Formazanfarbstoffen, die in Form der freien Säure der Formel I 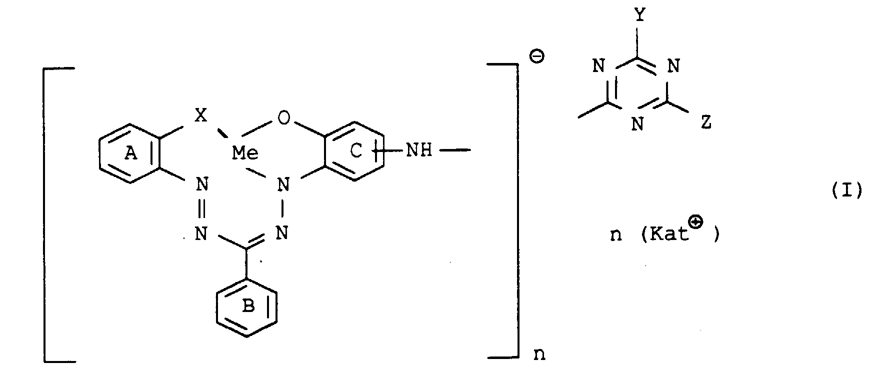 entsprechen, in der
Kat^{⊕} das Äquivalent eines Kations,
n 1 oder 2,
X Sauerstoff oder ein Rest der Formel CO-O oder SO₂-O,
Me Kupfer oder Nickel,
Y Halogen und
Z, wenn n 1 ist, Halogen, C₁-C₄-Alkoxy, Amino, C₁-C₄-Mono- oder Dialkylamino, substituiertes C₂-C₄-Alkylamino oder Phenyl- oder N-(C₁-C₄-Alkyl)-N-phenylamino, wobei die Phenylgruppe jeweils substituiert sein kann, oder wenn n 2 ist, einen Rest der Formel 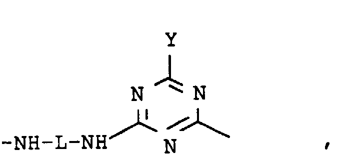 worin L für C₂-C₆-Alkylen oder gegebenenfalls durch Hydroxysulfonyl substituiertes Phenylen steht und Y die obengenannte Bedeutung besitzt, bedeuten und
die Ringe A, B und C weitere Substituenten tragen können,
dadurch gekennzeichnet, daß man
a) in einem 1. Schritt ein Aminophenol der Formel II 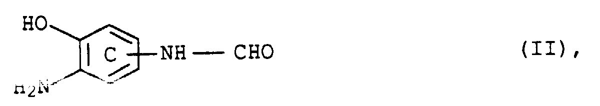 in der der Ring C die obengenannte Bedeutung besitzt, diazotiert, mit einer Kupplungskomponente der Formel III 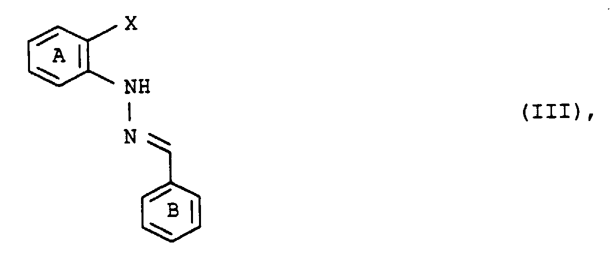 in der X und die Ringe A und B jeweils die obengenannte Bedeutung besitzen, kuppelt und vor, während oder nach der Kupplungsreaktion ein Kupfer- oder Nickelsalz zusetzt,
b) in einem 2. Schritt die resultierende Formazanverbindung der Formel IV 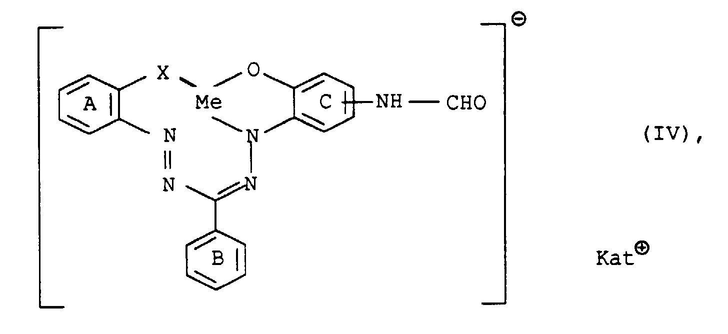 in der Kat^{⊕} X, Me und die Ringe A, B und C jeweils die obengenannte Bedeutung besitzen, säure- oder basenkatalysiert verseift und
c) in einem 3. Schritt die resultierende Aminoverbindung mit einem Triazin der Formel Va oder Vb 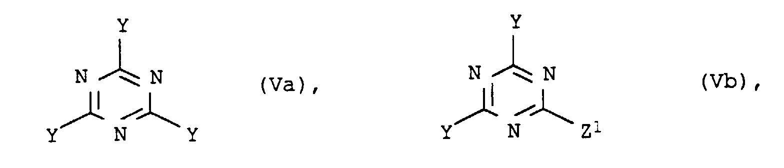 wobei Y jeweils die obengenannte Bedeutung besitzt und Z¹ C₁-C₄-Alkoxy, Amino, C₁-C₄-Mono- oder Dialkylamino, substituiertes C₂-C₄-Alkylamino oder Phenyl- oder N-(C₁-C₄-Alkyl)-N-phenylamino, wobei die Phenylgruppe jeweils substituiert sein kann, oder einen Rest der Formel 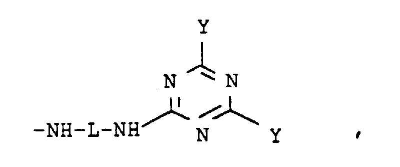 worin L und Y jeweils die obengenannte Bedeutung besitzen, bedeutet, zur Reaktion bringt.

2. Verfahren nach Anspruch 1, dadurch gekennzeichnet, daß in Formel I zwei der Ringe A, B und C jeweils einfach durch Hydroxysulfonyl substituiert sind.

3. Verfahren nach Anspruch 1, dadurch gekennzeichnet, daß Me Kupfer bedeutet.

4. Aminophenole der Formel II 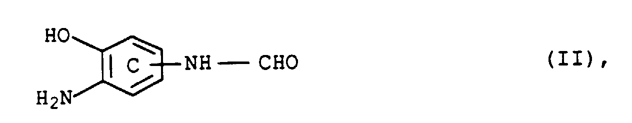 in der der Ring C substituiert sein kann.

## Claims

1. A process for preparing fiber-reactive formazan dyes which in the form of the free acid conform to the formula I 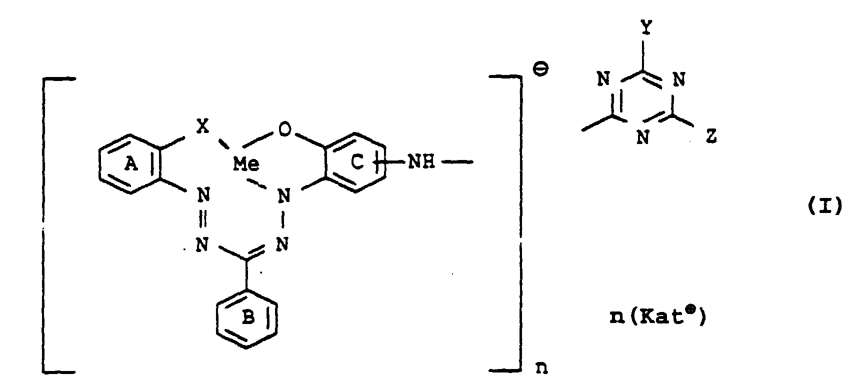 where
Kat^{⊕} is one equivalent of a cation,
n is 1 or 2,
X is oxygen or a radical of the formula CO-O or SO₂-O,
Me is copper or nickel,
Y is halogen,
Z for n=1 is halogen, C₁-C₄-alkoxy, amino, mono- or di (C₁-C₄-alkyl) amino, substituted C₂-C₄-alkylamino or phenyl- or N-(C₁-C₄-alkyl)-N-phenyl-amino, wherein the phenyl group may in each case be substituted, or for n=2 is a radical of the formula 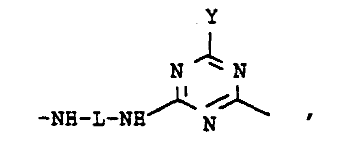 where L is C₂-C₆-alkylene or unsubstituted or hydroxysulfonyl-substituted phenylene and Y is as defined above, and
the rings A, B and C may carry further substituents, which comprises
a) as step 1 diazotizing an aminophenol of the formula II 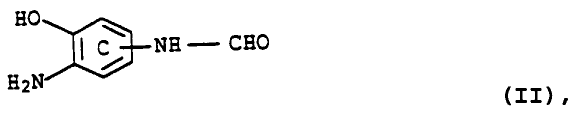 where the ring C is as defined above, coupling with a coupling component of the formula III 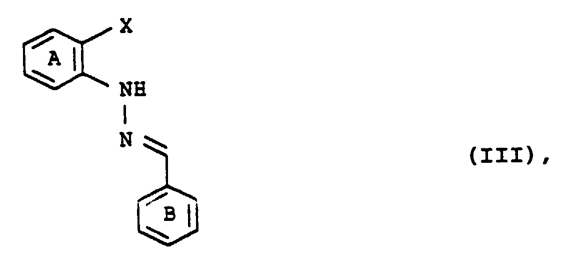 where X and the rings A and B are each as defined above, and before, during or after the coupling reaction adding a copper or nickel salt,
b) as step 2 hydrolyzing the resulting formazan compound of the formula IV 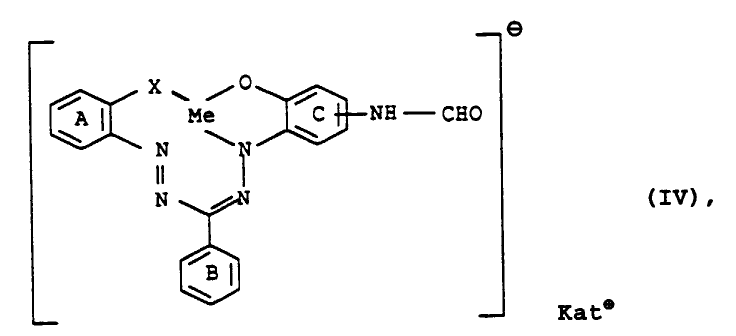 where Kat^{⊕}, X, Me and the rings A, B and C are each as defined above, under acid or base catalysis, and
c) as step 3 reacting the resulting amino compound with a triazine of the formula Va or Vb 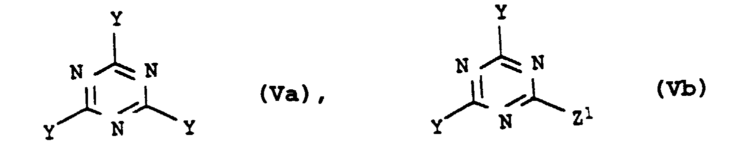 where Y is in either case as defined above and Z¹ is C₁-C₄-alkoxy, amino, mono- or di(C₁-C₄-alkyl)amino, substituted C₂-C₄-alkylamino, phenyl- or N-(C₁-C₄-alkyl)-N-phenyl-amino, wherein the phenyl group may in either case be substituted, or a radical of the formula 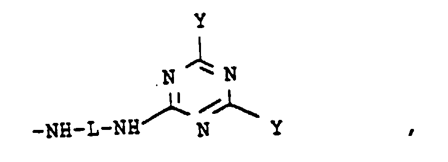 where L and Y are each as defined above.

2. A process as claimed in claim 1, wherein in formula I two of the rings A, B and C are each monosubstituted by hydroxysulfonyl.

3. A process as claimed in claim 1, wherein Me is copper.

4. Aminophenols of the formula II 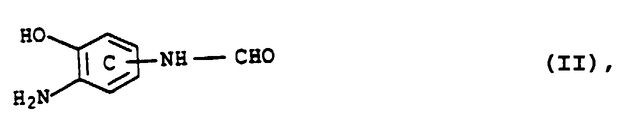 where the ring C may be substituted.

## Revendications

1. Procédé de fabrication de colorants de type formazan réagissant sur des fibres, qui correspondent, sous la forme de l'acide libre, à la formule I 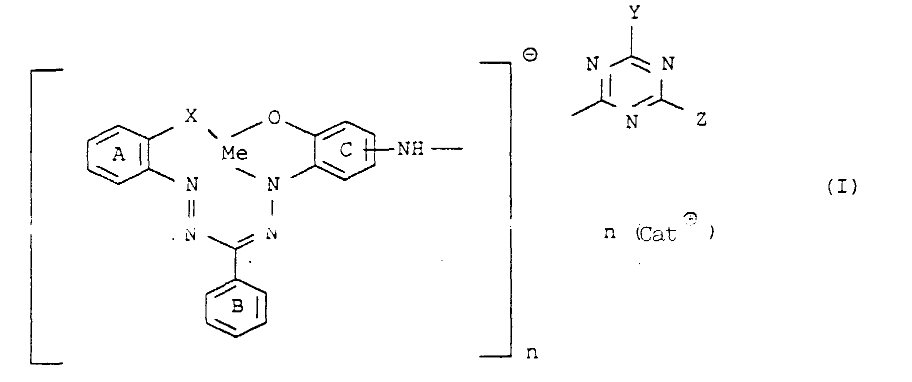 où
Cat^{⊕} signifie l'équivalent d'un cation,
n signifie 1 ou 2,
X signifie un atome d'oxygène ou un groupement de formule CO-O OU SO₂-O,
Me signifie un atome de cuivre ou de nickel,
Y signifie un atome d'halogène et
Z signifie, lorsque n vaut 1, un atome d'halogène, un groupement alcoxy en C₁-C₄, amino, mono- ou dialkylamino en C₁-C₄, alkylamino en C₂-C₄ ou phényl- ou N-(alkyle en C₁-C₄)-N-phénylamino substitué, où le groupement phényle peut être à chaque fois substitué, ou lorsque n vaut 2, un groupement de formule 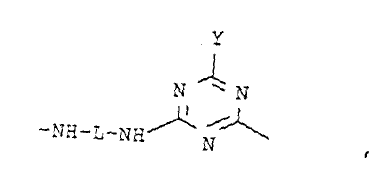 où L est mis pour un groupement alkylène en C₂-C₆ ou un groupement phénylène éventuellement substitué par un groupement hydroxysulfonyle et Y prend la signification susmentionnée, et
les cycles A, B et C peuvent porter d'autres substituants,
caractérisé en ce que
a) dans une première étape, on diazote un aminophénol de formule II 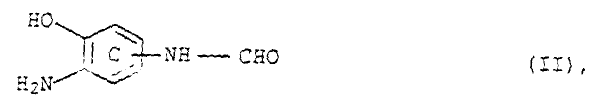 où le cycle C prend la signification susmentionnée, on le copule avec un composant de copulation de formule III 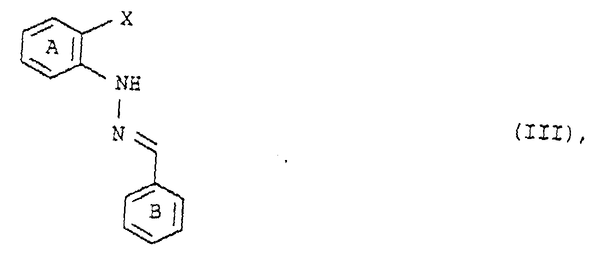 où X et les cycles A et B prennent chacun la signification susmentionnée, et l'on ajoute un sel de cuivre ou de nickel avant, pendant ou après la réaction de copulation,
b) dans une deuxième étape, on saponifie avec catalyse acide ou basique le composé de type formazan résultant de formule IV 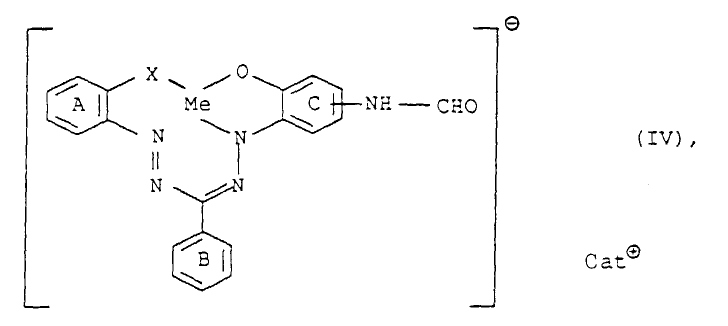 où Cat^{⊕}, X, Me et les cycles A, B et C prennent chacun la signification susmentionnée et
c) dans une troisième étape, on met en réaction le composé amino résultant avec une triazine de formule Va ou Vb 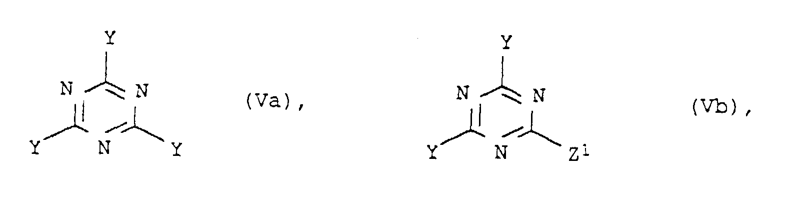 où Y prend à chaque fois la signification susmentionnée et Z¹ signifie un groupement alcoxy en C₁-C₄, amino, mono- ou dialkylamino en C₁-C₄, alkylamino en C₂-C₄ ou phényl- ou N-(alkyle en C₁-C₄)-N-phénylamino substitué, où le groupement phényle peut à chaque fois être substitué, ou un groupement de formule 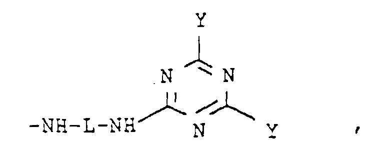 où L et Y prennent chaque fois la signification susmentionnée.

2. Procédé selon la revendication 1, caractérisé en ce que, dans la formule I, deux des cycles A, B et C sont substitués chacun une fois par un groupement hydroxysulfonyle.

3. Procédé selon la revendication 1, caractérisé en ce que Me signifie un atome de cuivre.

4. Aminophénols de formule II 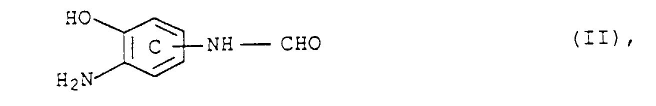 où le cycle C peut être substitué.
